# EUROPEAN PATENT SPECIFICATION

(11) **EP 0 343 714 B1**
(45) Date of publication and mention of the grant of the patent: **02.11.1994**
(21) Application number: 89201236.0
(22) Date of filing: 17.05.1989
(51) Int. Cl.: C12P 41/00, C12P 17/02, C07D 303/48, C07D 281/10

(54) **Phenylglycidate stereoisomers, conversion products thereof with e.g. 2-nitrothiophenol and preparation of diltiazem**
Phenylglycidat-Stereoisomere, Umsetzungsprodukte mit 2-Nitrothiophenol und Herstellung von Diltiazen
Stéréoisomères de phénylglycidate, produits de conversion de ceux-ci avec 2-nitrothiophénol et préparation de diltiazème

(30) Priority: 20.05.1988 NL 8801311
(43) Date of publication of application: 29.11.1989
(73) Proprietor: DSM N.V., 6411 TE Heerlen (NL)
(72) Inventor: Hulshof, Lumbertus Albregt, NL-5991 MA Baarlo (NL); Roskam, Jan Hendrik, NL-5462 CM Veghel (NL)

(56) References cited:
- EP-A- 0 059 335
- EP-A- 0 158 339
- EP-A- 0 237 983
- EP-A- 0 264 457
- EP-A- 0 342 902
- EP-A- 0 342 903
- EP-A- 0 342 904
- Chem.Abstr.108, 1988, 702, no.131290r

## Description

The present invention relates to a process for the preparation of enantiomerically pure transesters of phenylglycidic acids of the general formula: 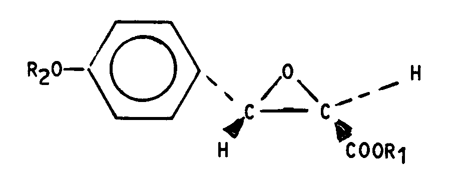
in which R₁ represents an alkyl radical and R₂ a hydrogen atom or an alkyl radical.

The invention also relates to the above process including the further conversion of such esters with 2-nitrothiophenol, 2-aminothiophenol or 2-(β-N,N-dimethylaminoethylamino)thiophenol. More in particular this aspect of the invention relates to the conversion of esters of (2R,3S)-3-(4-hydroxy- or 4-alkoxyphenyl)glycidic acid with 2-nitrothiophenol into esters of (2S,3S)-2-hydroxy-3-(4-hydroxy- or 4-alkoxyphenyl, respectively)-3-(2-nitrophenylthio)propionic acid. When using 2-aminothiophenol or 2-(β-N,N-dimethylaminoethylamino)thiophenol as starting material in this conversion the corresponding propionic acid derivatives are obtained.

The invention also relates to the use of the propionic acid derivatives so obtained for the preparation of diltiazem and to the preparation of diltiazem from such compounds, comprising the above processes.

Diltiazem is the name commonly used for 2-(4′-methoxyphenyl)-3-acetyloxy-5-[2-(dimethylamino)ethyl]-2,3-dihydro-1,5-benzo- thiazapin-4(5H)-one and is known from, for example, US-A-3.562.257. Diltiazem is a highly active coronary vasodilator and is used as such in medicine.

It is known that with biologically active compounds, such as pharmaceuticals, the spatial molecular structure is of great importance and can have a considerable influence on the desired activity. In a number of cases it has even been found that one or more stereoisomers of a biologically active compound do not only lack the desired activity or have insufficient activity, but moreover have a harmful effect. Because of this, increasingly stricter requirements are imposed regarding the purity of medicines and other biologically active compounds and, in particular, it is more and more often required that compounds that are used for medicinal and other such purposes are composed exclusively or virtually exclusively of one stereoisomer. In the framework of the present invention compounds consisting entirely or largely, i.e. more than 95 percent, of one stereoisomer are called stereoisomerically pure. To avoid misunderstanding, it should be noted that such a stereoisomer need not be pure otherwise and may even be greatly contaminated. Of course, for producing a stereoisomerically pure compound the efforts will be directed at the stereoisomer which most displays the desired activity and/or presents the least side-effects.

Of diltiazem the (2S,3S) isomer appeared the most effective and therefore that isomer is the most desired. For medicinal purposes the stereoisomer must be pure, that is, as free of the other stereoisomers as possible.

For the preparation of diltiazem use is usually made of a methoxyphenylglycidate that is coupled with amino-, β-N,N-dimethylaminoethylamino- or nitrothiophenol, after which the coupling product is converted into diltiazem in a number of steps. According to these syntheses mixtures of stereoisomers of diltiazem are obtained. The yields of multi-step processes are usually rather limited and this is also the case with the synthesis of diltiazem. Moreover, the yield of the desired (2S, 3S) isomer is at most half of the isomer mixture. Such a process is uneconomical on account of the amount of chemicals, solvents, catalysts, energy, etc. consumed in the preparation of the undesired isomer(s) and the associated burden on the environment. In addition, the installations must be unnecessarily large to be able to also produce the undesired isomers. Finally, it is a disadvantage that the expensive compound cinchonidine (Japanese Pat. Pub. No. 49-36221) must be used for the separation of the stereoisomers of diltiazem. It is therefore very desirable to be able to direct the preparation process of diltiazem towards the desired isomer already in the starting phase of the process. So far, a preparation process for diltiazem which is directed towards stereoisomerically pure compounds already in the starting phase is known only from US-A-4.552.695.

According to US-A-4.552.695 a derivative of trans-cinnamyl alcohol is epoxydized asymmetrically, which results in an optically active epoxy alcohol. This alcohol is oxidized into an acid and the acid is esterified. After this the oxirane ring is opened with hydrogen chloride and then the chlorohydrin formed is converted with o-nitrothiophenol, the chlorine atom in the chlorohydrin being replaced by the o-nitrothiophenol radical.

The process according to US-A-4.552.695 is time-consuming. First acetoxy-trans-cinnamyl alcohol is prepared by reducing acetoxy-trans-cinnamic acid, which is in its turn prepared from hydroxy-trans-cinnamic acid. After the asymmetrical epoxidation of acetoxy-trans-cinnamyl alcohol the epoxidized alcohol is oxidized into an acid, which acid is subsequently esterified. In a later phase the acetoxy group must be removed by deacylation and then the OH group must be converted into the methoxy group. The acid that is formed in the oxidation of the epoxidized alcohol is a 2,3-epoxypropionic acid. It is known that such acids are rather unstable and that they easily decarboxylate, which adversely affects the yield.

It has now been found that stereoisomerically pure esters of phenylglycidic acid derivatives of the general formula: 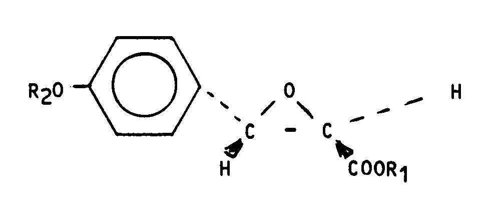
in which R₁ represents an alkyl radical and R₂ a hydrogen atom or an alkyl radical can easily be prepared by enzymatically hydrolyzing a mixture of the stereoisomers of esters of trans-phenylglycidic acid derivatives of the aforementioned general formula with the aid of a suitable lipase, peptidase or a proteolytic bacterial enzyme and isolating the non-hydrolyzed ester from the reaction mixture.

Surprisingly, it has been found that the (2S,3R) isomer in the stereoisomer mixture is enzymatically hydrolyzed into the corresponding acid and that the (2R,3S) isomer is not hydrolyzed. If, to prepare diltiazem, the aforementioned (2R,3S) ester is caused to react with 2-nitrothiophenol, 2-aminothiophenol or 2-(β-N,N-dimethylaminoethylamino)thiophenol under such conditions as to result in cis-opening of the oxirane ring, a (2S,3S) reaction product is obtained that can easily be processed further into the (2S,3S) isomer of diltiazem, which is the most desirable isomer.

The alkyl esters of trans-phenylglycidic acid derivatives of the aforementioned general formula can easily be prepared by Darzen's condensation of aromatic aldehydes with esters of chloroacetic acid, for example, anisaldehyde with methylchloroacetate, or by epoxydation of esters of hydroxy- or alkoxy-trans-cinnamic acid.

The condensation of trans-phenylglycidates with 2-nitrothiophenol in such a manner as to result in cis-opening of the oxirane ring is described in EP-A-59.335 and in an article by Hashiyama et al. in J. Chem. Soc. Perkin Trans. I (1984) 1725-1732. There is no mention of a preceding separation of the (2R, 3S) and (2S,3R) isomers of the trans-glycidic acid ester, which means that the use of these processes for the preparation of diltiazem also leads to a mixture of stereoisomers. Similarly, condensation reactions with 2-aminothiophenol have been described by Hashiyama et al. in J. Chem. Soc. Perkin Trans. I (1985) 421-427, and with 2-(β-N,N-dimethylaminoethylamino)thiophenol by Kugita et al. in Chem. Pharm. Bull. 19 (3), p. 595-602 (1971).

According to US-A-4.533.748, dl-2-hydroxy-3-(4-methoxyphenyl)-3-(2-aminophenylthio)propionic acid, which is a suitable intermediate for the synthesis of pharmacologically active compounds, in particular benzothiazepines, such as diltiazem, is separated into its enantiomers after salt formation with L-lysine.

Enzymatic hydrolysis of esters, as used according to the present invention for the hydrolysis of the corresponding glycidate, is commonly known. Proteolytic bacterial enzymes, peptidases and lipases may be used for this purpose. Proteolytic bacterial enzymes and peptidases are protein-splitting enzymes that can be obtained from yeasts, fungi and animal species. They are produced on a technical scale and are commercially available. Lipases are fat-splitting enzymes that can be bacterially recovered from yeast or fungi species, pig's liver, etc. Lipases are also commercially available.

The enzymatic hydrolysis of the glycidates discussed here is preferably carried out in a mixture of water and an organic solvent, such as water-methyl-tert.-butylether. The hydrolysis may be effected at ambient temperature (or even as low as -10¤C), or at slightly higher temperatures, up to about 60¤C. During the hydrolysis the pH is maintained at a constant value in the range from 3 to 11, preferably at about 5 to 8, by the addition of a base. The specific conditions, of course, vary with the enzyme used. One skilled in the art can easily determine which enzymes and which conditions give good results. When no more base is consumed the hydrolysis is completed and the residual ester, that is, the stereoisomer that has not been hydrolyzed, is recovered by removing the organic solvent in which the ester is dissolved and isolating the ester from that solution. If so desired, the ester may be purified by recrystallization or in some other known manner.

The enzymes, of course, also may be immobilized or enclosed in a membrane, thereby facilitating re-use of the enzyme, continuous processing, separation of layers etcetera.

According to the present invention a stereoisomerically pure ester of (2R, 3S)-3-(4-hydroxy or 4-alkoxyphenyl)-glycidic acid is obtained in this manner. Preferably, an ester of (2R,3S)-3-(4-methoxyphenyl)-glycidic acid is prepared in this manner. This ester is then allowed to couple with 2-nitrothiophenol in the presence of a Lewis acid, preferably in the presence of a tin(II) compound. In the presence of a Lewis acid, in particular a tin(II) compound, the coupling can be effected in such a manner as to result in exclusively cis-opening of the oxirane ring, so that exclusively or almost exclusively an ester of (2S,3S)-2-hydroxy-3-(4-methoxy-phenyl)-3-(2-nitrophenylthio)propionic acid is obtained which can then be converted into the desired (2S,3S) isomer of diltiazem in a known manner. When 2-aminothiophenol or 2-(β-N,N-dimethylaminoethylamino)thiophenol is used instead of 2-nitrothiophenol the corresponding propionic acid derivatives are obtained, which in turn can be converted into the desired isomer of diltiazem.

With the present invention it is now possible to limit the synthesis of diltiazem already at an early stage to those stereoisomers - to the exclusion of other possible stereoisomers - which eventually yield the (2S,3S) diltiazem.

According to the present invention it is now possible to prepare the (2S, 3S) isomer of diltiazem - to the exclusion of other stereoisomers - by enzymatically hydrolyzing a mixture of stereoisomers of an ester of a derivative of trans-phenylglycidic acid, in particular the 3-(4-methoxyphenyl) derivative of trans-phenylglycidic acid of the aforementioned formula, with the aid of a suitable lipase, peptidase or a proteolytic enzyme, recovering the non-hydrolyzed ester from the reaction mixture, and coupling this ester with e.g. 2-nitrothiophenol under such conditions as to result in cis-opening of the oxirane ring. Then - if 2-nitrothiophenol is used - the nitro group of the coupling product thus obtained is reduced into the amino group in a known manner, after which, if so desired, or if necessary after the introduction of protective groups according to the methods usually applied for the formation of amides, ring closure into a thiazepine ring is effected. Then the dimethylaminioethyl group and the acetoxy group are fed to the nitrogen atom of the thiazepine ring and to the 3-hydroxy group, respectively, in a known manner (in whatever order). When starting from 2-aminothiophenol instead of 2-nitrothiophenol this reaction sequence is shortened, and even more so if 2-(β-N,N-dimethylaminoethylamino)thiophenol is used instead of 2-nitrothiophenol.

The ivention is further elucidated with the following examples without, however, being limited thereby.

Examples I-III describe screenings with commercially available enzymes.

### Example I

### Screening of enzyme preparations on racemic trans-methyl(p-methoxyphenyl)glycidate

2.0 g of racemic trans-methyl(p-methoxyphenyl)glycidate was dissolved in 6 ml of toluene. To this solution 6 ml of a potassium-phosphate buffer solution (pH = 7.8, 50 mM) was added. To the stirred mixture a quantity of enzyme as indicated in Table 1 was added. With the aid of automatic titration with 0.1 N NaOH the pH was maintained at 7.8. After 24 hours a sample of the reaction mixture was taken and with the aid of HPLC (column: chiralcel OD; eluent: hexane/isopropanol = 90/10) the enantiomeric excess (e.e.) was determined.

The results have been summarized in Table 1.

**TABLE 1**

| Enzyme | Quantity | e.e. (after 24 hours) |
|---|---|---|
| Bacillus licheniformis | 200 µl | 16 |
| Bacillus subtilis | 100 mg | 16 |
| α-Chymotrypsin | 100 mg | 0 |
| Streptomyces griseus | 200 mg | 0 |
| Acylase I | 200 mg | 0 |
| Pen-V-acylase | 640 mg | 14 |
| Bacterial proteinase | 200 µl | 0 |
| Aspergillus oryzae | 100 mg | 6 |
| Bovine pancreas | 100 µl | 0 |
| Pregastric lipase | 200 mg | 0 |
| Rhizopus javanices | 200 mg | 5 |
| Porcine pancreatic lipase | 250 mg | 15 |
| Mucor javanicus | 100 mg | 34 |
| Candida cylindraceae | 250 mg | 47 |
| Aspergillus niger | 200 mg | 0 |
| Rhizopus niveus | 200 mg | 10 |
| Rhizopus delemar | 200 mg | 21 |
| Mucor miehei | 200 mg | 100 |
| Pseudomonas fluorescens | 260 mg | 81 |
| Lipoprotein lipase | 40 mg | 51 |
| Geotrichum candidum | 200 mg | 0 |
| Penicillium cyclopium | 200 mg | 0 |

### Example II

### Screening of enzyme preparations on racemic trans-ethyl(p-methoxyphenyl)glycidate

45 g of racemic trans-ethyl(p-methoxyphenyl)glycidate was dissolved in 135 ml of toluene. From this solution 5.5 ml portions were successively taken, to which 9.5 ml of a potassium phosphate buffer solution (pH = 7.8, 50 mM) was added. To this mixture, which was kept stirred, a quantity of enzyme was added as indicated in table 2, and the pH was maintained at 7.8 with the aid of automatic titration with 0.1 N NaOH. After 48 hours a sample of the reaction mixture was taken, which then was analyzed with the aid of HPLC (chiralcel OD; hexane/ isopropanol = 90/10) for determining the enantiomeric excess.

The results have been summarized in Table 2.

**TABLE 2**

| Enzyme | Quantity | e.e. (after 48 hours) |
|---|---|---|
| Bacillus licheniformis | 200 µl | 19 |
| α-Chymotrypsin | 50 mg | 0 |
| Mucor javanicus | 31 mg | 11 |
| Rhizopus delemar | 51 mg | 0 |
| Wheat germ lipase | 95 mg | 0 |
| Mucor miehei | 200 µl | 48 |
| Aspergillus niger | 15 mg | 8 |
| Pseudomonas fluorescens | 43 mg | 63 |
| Chromobacterium viscosum | 0.5 mg | 6 |
| Porcine pancreatic lipase | 200 mg | 0 |

### Example III

### Screening of enzyme preparations on racemic trans-butyl(p-methoxyphenyl)glycidate

1.0 g of racemic trans-butyl(p-methoxyphenyl)glycidate was dissolved in 3 ml of toluene. To this solution 3 ml of the same buffer as used in the previous Examples was added. The mixture was stirred and, after addition of a quantity of lipase enzyme as indicated in Table 3, was kept at pH 7.8 with the aid of automatic titration with 0.1 N NaOH. After 40 hours a sample of the reaction mixture was taken and with the aid of HPLC (chiralcel OD; hexane/isopropanol = 96/4) the enantiomeric excess was determined.

The results have been summarized in Table 3.

**TABLE 3**

| Enzyme | Quantity | e.e. (after 40 hours) |
|---|---|---|
| Mucor miehei | 32 mg | 40 |
| Aspergillus niger | 26 mg | 11 |
| Chromobacterium viscosum | 32 mg | 27 |
| Pseudomonas fluorescens | 88 mg | 54 |
| Mucor javanicus | 100 mg | 18 |
| Porcine pancreatic lipase | 98 mg | 0 |
| Wheat germ lipase | 100 mg | 0 |

Thus, from screenings like those in the foregoing - which were all done with commercially available enzymes - it is concluded that one skilled in the art can easily determine which of the proteolytic bacterial enzymes, lipases and peptidases are suitable for the process according to the present invention. Of course, also suitable proteolytic bacterial enzymes, lipases and peptidases which are not commercially available may be used.

Through this screening, enzymes can be selected which show the desired selectivity towards the substrate used. Where the e.e. is found to be zero the enzyme is not suitable for the enzymatic conversion according to the present invention.

The above screenings clearly show that suitable proteolytic bacterial enzymes, lipases and peptidases exist with the enantio-selective hydrolytic activity according to the invention.
Best results are found in the group of lipases.

It should be noted here that the results from the screening experiments have not been optimized as to specific reaction conditions such as pH, temperature, concentrations of substrate and enzyme, solvent, etcetera. Neither were the reactions continued up to maximum conversion. This can be easily established by one skilled in the art.

The following example describe preparations of (2R,3S)-p-methoxyphenylglycidate esters.

### Example IV

### Preparation of (2R, 3S)-methyl(p-methoxyphenyl)glycidate

90 g of racemic trans-methyl(p-methoxyphenyl)glycidate was dissolved in 900 ml of methyl-tert.-butylether (TBME) at 30¤C. Then 900 ml of 50 mM Tris-HCl buffer with a pH of 7.8 was added.
After addition of 30 ml of Bacillus protease the pH was maintained at 7.8 with the aid of automatic titration with 5 N NaOH.

When the lye consumption had dropped to zero, the stirring was stopped and the layers were separated. The water layer was extracted two more times with 300 ml of TBME and the collected TBME phase was washed 3x with 300 ml of 20% Na-bisulphite solution and then once more with 5% Na-bicarbonate solution. The TBME was dried over MgSO4 and evaporated. 44.2 g of light yellow crystals (49%) was obtained.
[α]²⁰_{D} = -150.5¤ (C=1, MeOH)
By recrystallization from 45 ml of ethylacetate 26.2 g (29.2%) of white crystals was obtained
[α]²⁰_{D} = -205¤ (C=1, MeOH). Melting temperature 84¤-87¤C.

### Example V

Example IV was repeated on the understanding that methyl isobutyl ketone was used as solvent instead of methyl-tert.-butylether, which resulted in 43 g of crystals, which, after recrystallization from ethyl acetate, produced 25.5 g of white crystals with the same purity as obtained in example IV.

### Example VI

### Preparation of (2R,3S)-ethyl(p-methoxyphenyl)glycidate

100 g of racemic trans-ethyl(p-methoxyphenyl)glycidate was dissolved in 500 ml of toluene at 30¤C. To this solution 500 ml of 50 mM Tris-HCl buffer with a pH of 7.8 was added.
After the addition of 20 ml of Mucor miehei lipase the pH was maintained while stirring at 7.8 with the aid of automatic titration with 5 N NaOH.
When the lye consumption had dropped to zero, the stirring was stopped and the layers were separated. The water layer was extracted two more times with 300 ml of toluene and the collected toluene phase was washed 3 times with 300 ml of a 20% aqueous solution of Na-bisulphite and then with 5% Na-bicarbonate solution, to neutral. The toluene phase then was dried over MgSO₄ and evaporated.
The remaining product was analyzed by means of HPLC techniques (chiralcel OD; hexane/isopropanol = 90/10) and an e.e. of 99% was found.

### Example VII

### Preparation of (2R,3S)-butyl(p-methoxyphenyl)glycidate

100 g of racemic trans-butyl(p-methoxyphenyl)glycidate was mixed while stirring with 150 ml of 50 mM Tris.HCl buffer, pH = 7.8.
After the addition of 5 ml of Mucor miehei lipase the pH was maintained while stirring at 7.8 with the aid of automatic titration with 5 N NaOH.
When the lye consumption had dropped to zero the stirring was stopped and the layers were separated.
The water layer was extracted 2 times with 300 ml of toluene and the combined organic phase then was extracted 3 times with 100 ml of a 20% aqueous solution of Na-bisulphite and then with 5% Na-bicarbonate solution, to neutral. The organic phase then was dried over MgSO₄ and evaporated.
A slightly yellow oil was obtained.
According to HPLC determination (chiralcel OD; hexane/isopropanol = 96/4) an e.e. of 99% was found.

## Claims

1. Process for the preparation of stereoisomerically pure esters of phenylglycidic acids of the general formula: 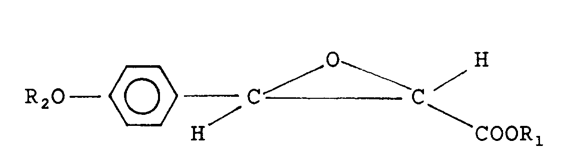 in which R₁ represents an alkyl radical and R₂ a hydrogen atom or an alkyl radical, characterized in that a mixture of the stereoisomers of esters of trans-phenylglycidic acid derivatives of the aforementioned general formula is enzymatically, enantioselectively hydrolyzed with the aid of a suitable enzyme and the non-hydrolyzed (2R,3S) ester is isolated from the reaction mixture.

2. Process according to claim 1, wherein R₂ is methyl.

3. Process according to claim 2, wherein R₁ is methyl.

4. Process for the preparation of stereoisomerically pure esters of 2-hydroxy-3-(4-hydroxy or 4-alkoxyphenyl)-3-[(2-nitrophenylthio), (2-aminophenylthio) or (2-β-N,N-dimethylaminoethylamino) phenylthio]-propionic acid, characterized in that a mixture of the stereoisomers of an ester of a phenylglycidic acid of the general formula 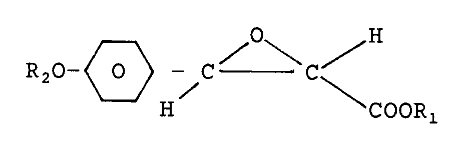 in which R₁ represents an alkylradical and R₂ a hydrogen atom or an alkyl radical is enzymatically, enantioselectively hydrolyzed with the aid of a suitable enzyme, the non-hydrolyzed (2R, 3S) ester is isolated from the reaction mixture and coupled with 2-nitrothiophenol, 2-aminothiophenol or 2-(β-N,N-dimethylamino-ethylamino)thio-phenol under such conditions as to result in cis-opening of the oxirane ring.

5. Process for the preparation of (2S,3S)-2-(4'-methoxyphenyl)-3-acetyloxy-5-[2- (dimethylamino)ethyl]-2,3-dihydro-1,5-benzothiazepin-4(5H)-one, characterized in that a mixture of the stereoisomers of an ester of a phenylglycidic acid of the general formula 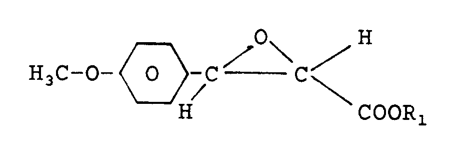 in which R₁ represents an alkyl radical, is enzymatically, enantioselectively hydrolyzed with the aid of a suitable enzyme, the non-hydrolyzed (2R,3S)-ester is isolated from the reaction mixture and coupled with 2-nitrothiophenol, 2-aminothiophenol or 2-(β-N,N-dimethylamino-ethyl-amino)thiophenol under such conditions as to result in cis-opening of the oxirane ring, after which the coupling product thus obtained is further converted in a known manner to (2S,3S)-2-(4'-methoxyphenyl)-3-acetyloxy-5-[2-(dimethylamino)ethyl]-2,3-dihydro-1,5-benzothiazepin-4-(5H)-one.

6. Process according to any one of claims 1-5 wherein as an enzyme, a lipase, peptidase or a proteolytic bacterial enzyme is used.

7. Process according to claim 6, wherein the enzymatic hydrolysis is performed with the aid of a suitable lipase.

8. Process according to anyone of claims 1-7, wherein the mixture of stereoisomers of trans-phenylglycidic acid derivatives is dissolved in an organic solvent and the enzymatic hydrolysis is carried out in a two-phase system.

9. Process according to anyone of claims 1-8 wherein the non hydrolyzed (2R,3S)-ester of the trans-phenylglycidic acid derivative is purified.

10. Process according to claim 9 wherein the non hydrolyzed (2R,3S)-ester of the trans-phenylglycidic acid derivative is purified by recrystallization.

## Patentansprüche

1. Verfahren zur Herstellung stereoisomer reiner Ester von Phenylglycidsäure der allgemeinen Formel: 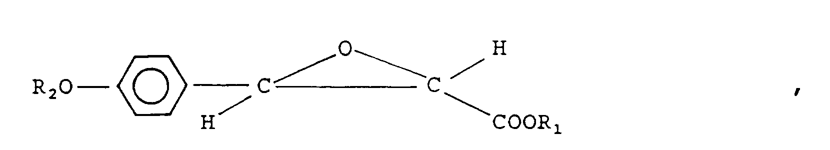 worin R₁ einen Alkylrest und R₂ ein Wasserstoffatom oder einen Alkylrest darstellt, dadurch gekennzeichnet, daß eine Mischung der Stereoisomere von Estern von trans-Phenylglycidsäure-Derivaten der oben genannten allgemeinen Formel mit Hilfe eines geeigneten Enzyms enzymatisch, enantioselektiv hydrolysiert wird, und der nicht-hydrolysierte (2R,3S)-Ester aus der Reaktionsmischung isoliert wird.

2. Verfahren nach Anspruch 1, wobei R₂ Methyl bedeutet.

3. Verfahren nach Anspruch 2, wobei R₁ Methyl ist.

4. Verfahren zur Herstellung stereoisomer reiner Ester von 2-Hydroxy-3-(4-hydroxy oder 4-alkoxyphenyl)-3-[(2-nitrophenylthio), (2-aminophenylthio) oder (2-β-N,N-dimethylaminoethylamino)-phenylthio]-propionsäure, dadurch gekennzeichnet, daß eine Mischung der Stereoisomere eines Esters einer Phenylglycidsäure der allgemeinen Formel 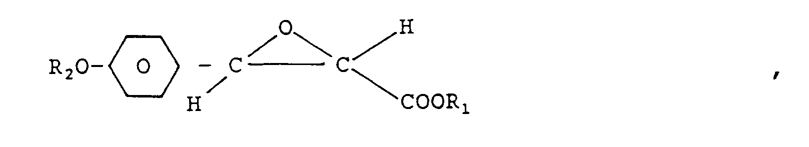 worin R₁ einen Alkylrest und R₂ ein Wasserstoffatom oder einen Alkylrest darstellt, mit Hilfe eines geeigneten Enzyms enzymatisch, enantioselektiv hydrolysiert wird, der nicht-hydrolysierte (2R,3S)-Ester aus der Reaktionsmischung isoliert wird und mit 2-Nitrothiophenol, 2-Aminothiophenol oder 2-(β-N,N-Dimethylaminoethylamino)-thiophenol unter derartigen Bedingungen gekoppelt wird, daß eine cis-Öffnung des Oxiranrings erhalten wird.

5. Verfahren zur Herstellung von (2S,3S)-2-(4'-Methoxyphenyl)-3-acetyloxy-5-[2-(dimethylamino)-ethyl]-2,3-dihydro-1,5-benzo thiazepin-4-(5H)-on, dadurch gekennzeichnet, daß eine Mischung der Stereoisomere eines Esters einer Phenylglycidsäure der allgemeinen Formel 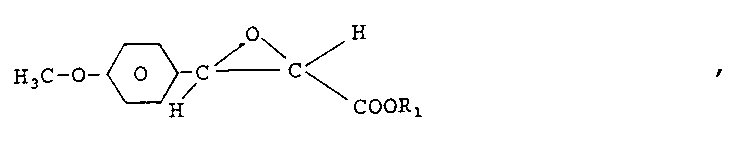 worin R₁ einen Alkylrest darstellt, mit Hilfe eines geeigneten Enzyms enzymatisch, enantioselektiv hydrolysiert wird, der nicht-hydrolysierte (2R,3S)-Ester aus der Reaktionsmischung isoliert wird und mit 2-Nitrothiophenol, 2-Aminothiophenol oder 2-(β-N,N-Dimethylaminoethylamino)-thiophenol unter derartigen Bedingungen gekoppelt wird, daß eine cis-Öffnung des Oxiranrings erhalten wird, wonach das so erhaltene Kopplungsprodukt auf bekannte Weise weiter in (2S,3S)-2-(4'-Methoxyphenyl)-3-acetyloxy-5-[2-(dimethylamino)-ethyl]-2,3-dihydro-1,5-benzothiazepin-4-(5H)-on übergeführt wird.

6. Verfahren nach einem der Ansprüche 1 bis 5, bei welchem als Enzym eine Lipase, Peptidase oder ein proteolytisches bakterielles Enzym verwendet wird.

7. Verfahren nach Anspruch 6, bei welchem die enzymatische Hydrolyse mit Hilfe einer geeigneten Lipase durchgeführt wird.

8. Verfahren nach einem der Ansprüche 1 bis 7, bei welchem die Mischung von Stereoisomeren von trans-Phenylglycidsäure-Derivaten in einem organischen Lösungsmittel gelöst wird, und die enzymatische Hydrolyse in einem Zweiphasen-System durchgeführt wird.

9. Verfahren nach einem der Ansprüche 1 bis 8, bei welchem der nicht-hydrolysierte (2R,3S)-Ester des trans-Phenylglycidsäure-Derivats gereinigt wird.

10. Verfahren nach Anspruch 9, bei welchem der nicht-hydrolysierte (2R,3S)-Ester des trans-phenylglycidsäure-Derivats durch Umkristallisation gereinigt wird.

## Revendications

1. Procédé de préparation d'esters stéréoisomériquement purs d'acides phénylglycidiques de formule générale : 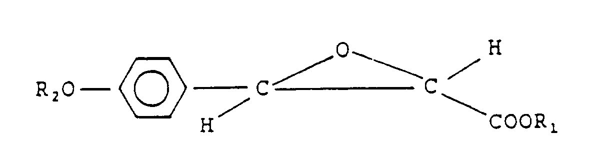 dans laquelle R₁ représente un radical alkyle et R₂ est un atome d'hydrogène ou un radical alkyle , caractérisé en ce qu'on hydrolyse énantiosélectivement par voie enzymatique un mélange de stéréoisomères des esters de dérivés d'acides trans phénylglycidiques de la formule générale précitée, à l'aide d'une enzyme appropriée et on isole l'ester non hydrolysé (2R,3S) du mélange de réaction.

2. Procédé selon la revendication 1, dans lequel R₂ est un radical méthyle.

3. Procédé selon la revendication 2, dans lequel R₁ est un radical méthyle.

4. Procédé de préparation d'esters stéréoisomériquement purs d'acides 2-hydroxy-3-(4-hydroxy- ou 4-alcoxyphényl)-3-((2-nitrophénylthio), (2-aminophénylthio) ou (2-bêta,N,N-diméthylaminoéthylamino)phénylthio)-propioniques, caractérisé en qu'on hydrolyse énantiosélectivement par voie enzymatique un mélange de stéréoisomères d'un ester d'acide phénylglycidique de formule générale : 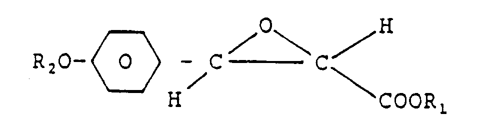 dans laquelle R₁ représente un radical alkyle et R₂ est un atome d'hydrogène ou on radical alkyle, à l'aide d'une enzyme appropriée, on isole l'ester non hydrolysé (2R,3S) du mélange de réaction et on le couple avec le 2-nitrothiophénol, le 2-aminothiophénol ou le 2-(bêta-N,N-diméthylamino-éthylamino)thiophénol dans des conditions aboutissant à une ouverture cis du noyau oxiranne.

5. Procédé de préparation de (2S,3S)-2-(4'-méthoxyphényl)-3-acétyloxy-5-(2-(diméthylamino)éthyl)-2,3-dihydro-1,5-benzothiazépin-4-(5H)-one, caractérisé en ce qu'or)hydrolyse énantiosélectivement par voie enzymatique un mélange de stéréuisomères d'un ester d'un acide phénylglycidique de formule générale : 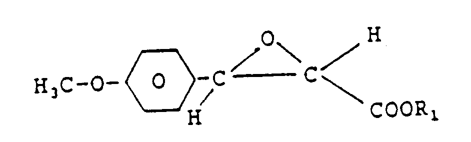 dans laquelle R₁ représente un radical alkyle, à l'aide d'une enzyme appropriée, on isole l'ester (2R,3S) non hydrolysé du mélange de réaction et on couple avec le 2-nitrothiophénol, le 2-aminothiophénol ou le 2-(bêta-N,N-diméthylamino-éthylamino)thiophénol dans des conditions telles qu'on obtient une ouverture cis du noyau oxiranne, après quoi on convertit le produit de couplage ainsi obtenu par une technique connue en (2S,3S)-2-(4'-méthoxyphényl)-3-acétyloxy-5-(2-(diméthylamino)éthyl)-2,3-dihydro-1-5-benzo-thiazépin-4-(5H)-one.

6. Procédé selon l'une quelconque des revendications 1 à 5, dans lequel on utilise une enzyme une lipase, peptidase ou une enzyme bactérienne protéolytique.

7. Procédé selon la revendication 6, dans lequel on effectue l'hydrolyse enzymatique à l'aide d'une lipase appropriée.

8. Procédé selon l'une quelconque des revendications 1 à 7, dans lequel on dissout un mélange de stéréoisomères de dérivés d'acides trans-phénylglycidiques dans un solvant organique et on effectue l'hydrolyse enzymatique dans un système à deux phases.

9. Procédé selon l'une quelconque des revendications 1 à 8, dans lequel on purifie l'ester non hydrolysé (2R,3S) du dérivé d'acide trans-phénylglycidique.

10. Procédé selon la revendication 9, dans lequel l'ester non hydrolysé (2R,3S) du dérivé d'acide trans-phénylglycidique est purifié par recristallisation.
